(19) Europäisches Patentamt
European Patent Office
Office européen des brevets

(11) Veröffentlichungsnummer: **0 102 542**
**B1**

(12) # EUROPÄISCHE PATENTSCHRIFT

(45) Veröffentlichungstag der Patentschrift: **04.07.90**

(21) Anmeldenummer: **83107784.7**

(22) Anmeldetag: **08.08.83**

(51) Int. Cl.$^5$: **C 07 D 417/12,** A 01 N 43/00, C 07 D 285/00

(54) **1,2,4,6-Thiatriazin-1,1-dioxid-ether, Verfahren zu ihrer Herstellung und ihre Verwendung zur Bekämpfung unerwünschten Pflanzenwuchses.**

(30) Priorität: **24.08.82 DE 3231394**

(43) Veröffentlichungstag der Anmeldung:
**14.03.84 Patentblatt 84/11**

(45) Bekanntmachung des Hinweises auf die Patenterteilung:
**04.07.90 Patentblatt 90/27**

(84) Benannte Vertragsstaaten:
**DE FR IT**

(56) Entgegenhaltungen:
**EP-A-0 039 426**

(73) Patentinhaber: **BASF Aktiengesellschaft**
**Carl-Bosch-Strasse 38**
**D-6700 Ludwigshafen (DE)**

(72) Erfinder: **Hamprecht, Gerhard, Dr.**
**Rote-Turm-Strasse 28**
**D-6940 Weinheim (DE)**
Erfinder: **Wuerzer, Bruno, Dr.Dipl.-Landwirt**
**Ruedigerstrasse 13**
**D-6701 Otterstadt (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 102 542 B1

Courier Press, Leamington Spa, England.

**Beschreibung**

Die vorliegende Erfindung betrifft 1,2,4,6-Thiatriazin-1,1-dioxid-ether, Verfahren zu ihrer Herstellung, Herbizide, die diese Verbindungen als Wirkstoffe enthalten, und ihre Verwendung zur Bekämpfung unerwünschten Pflanzenwuchses.

Aus der Literatur sind Thiatriazin-Verbindungen mit herbizider Wirkung bekannt. So werden beispielsweise in der DE—A 2 508 832 und in der DE—A 2 933 889 Thiatriazinondioxide mit folgendem Grundkörper

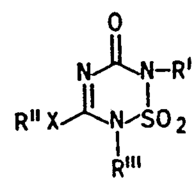

beschrieben

Desweiteren werden in der EP—A 39 426 und der US—A 4 343 648 u.a. 1,2,4,6-Thiatriazin-1,1-dioxidether der Formel I' beschrieben.

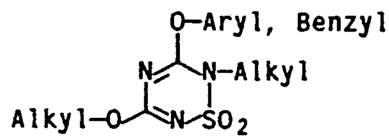

(I')

Die bekannten Verbindungen der genannten Art sind jedoch in vieler Hinsicht verbesserungsfähig, z.B. hinsichtlich Aufwandmenge und Selektivität, etwa bei nebeneinander vorkommenden grasartigen und breitblättrigen Pflanzen.

Gegenstand der vorliegenden Erfindung sind 1,2,4,6-Thiatriazin-1,1-dioxid-ether der Formel I

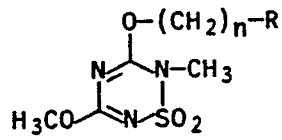

(I)

in der

R einen gegebenenfalls durch Alkyl mit 1 bis 4 Kohlenstoffatomen, Trifluormethyl, Nitro und/oder Halogen substituierten 6-gliedrigen aromatischen heterocyclischen Rest mit 1 oder 2 Stickstoffatomen, oder 1,2,3-Thiadiazol und

n 0 oder 1 bedeuten.

R in Formel I steht für einen gegebenenfalls durch Alkyl mit 1 bis 4 Kohlenstoffatomen, Trifluormethyl, Nitro oder Halogen substituierten 6-gliedrigen aromatischen heterocyclischen Rest mit 1 oder 2 Stickstoffatomen, z.B. für Reste folgender Heterocyclen: Pyridin, 2-, 3-, 4-Methylpyridin, 2-, 3-, 4-Chlorpyridin, 2,3,4-Trifluormethylpyridin, 2-, 3-, 4-Nitropyridin, 3-Chlor-5-trifluormethylpyridin, 3,5-Dichlorpyridin, 3,5-Dibromopyridin, 3,5-Diiodpyridin, Pyrimidin, 2-, 4-, 5-Methylpyrimidin, 2,6-Dimethylpyrimidin, 2-Isopropyl-6-methyl-pyrimidin, 2-Methyl-6-ethylpyrimidin, 2-, 4-, 5-Chlorpyrimidin, 2,4,5-Trifluor-methylpyridin, 2-, 4-, 5-Nitropyrimidin, 2,4-Dichlorpyrimidin, Pyrazin, 2-Methylpyrazin, 2-Trifluormethylpyrazin, 2-Nitropyrazin, 2-Chlorpyrazin, 2,5-Dichlorpyrazin, Pyridazin, 3-, 4-Methylpyridazin, 3,6-Dimethylpyridazin, 3-, 4-Chlorpyridazin, 3,6-Dichlorpyridazin oder 1,2,3-Thiadiazol, Bevorzugte Verbindungen der Formel I sind solche, bei denen R für einen unsubstituierten oder durch Methyl, Nitro oder Chlor substituierten Pyridinrest und n für 0 oder 1 stehen, oder solche, bei denen R für einen durch Alkyl mit 1 bis 4 Kohlenstoffatomen oder Halogen substituierten Pyrimidin-, oder 1,2,3-Thiadiazolrest, und n für 0 oder 1 stehen, oder solche bei denen R für einen Pyridyl-, Methylpyridyl-, Pyridyl-, 1,2,3-Thiadiazolyl-, oder einen 2-Isopropyl-6-methyl-pyrimidinylrest und n für 0 oder 1 stehen.

Man erhält die 1,2,4,6-Thiatriazin-1,1-dioxid-ether der Formel I durch Umsetzung von Verbindungen der Formel II

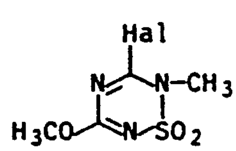

(II)

in der Hal Halogen bedeutet, mit einer Verbindung der Formel III

$$HO-(CH_2)_n-R \quad \text{(III)}$$

in der

R und n die obengenannten Bedeutungen haben, oder einem Alkali-, Erdalkali- oder Ammoniumsalz einer Verbindung der Formel III gegebenenfalls in einem inerten organischen Lösungsmittel und gegebenenfalls in Anwesenheit eines Säureakzeptors bei einer Temperatur zwischen −50 und +150°C. Die Umsetzung kann drucklos oder unter Druck kontinuierlich oder diskontinuierlich durchgeführt werden.

Zweckmäßigerweise verwendet man für die Umsetzung unter den jeweiligen Reaktionsbedingungen inerte Lösungs- oder Verdünnungsmittel. Als Lösungsmittel kommen beispielsweise Halogenkohlenwasserstoffe, insbesondere Chlorkohlenwasserstoffe, z.B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Jodbenzol, o-, m-, p-Dichlorbenzol, o-, p-, m-Dibrombenzol, o-, m-, p-Chlortoluol, 1,2,4-Trichlorbenzol; Ether, z.B. Ethylpropylether, Methyl-tert.-butylether, n-Butylethylether, Di-n-butylether, Di-isobutylether, Diisoamylether, Diisopropylether, Anisol, Phenetol, Cyclohexylmethylether, Diethylether, Ethylenglykoldimethylether, Tetrahydrofuran, Dioxan, Thioanisol, β,β'-Dichlordiethylether; Nitrokohlenwasserstoffe, wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester, z.B. Ethylacetat, Acetessigester, Isobutylacetat; Amide, z.B. Formamid, Methylformamid, Dimethylformamid; Ketone, z.B. Aceton, Methylethylketon, gegebenenfalls auch Wasser und entsprechende Gemische in Betracht. Zweckmäßigerweise verwendet man das Lösungsmittel in einer Menge von 100 bis 2.000 Gew.%, vorzugsweise von 200 bis 700 Gew.%, bezogen auf Ausgangsstoff II.

Als Säureakzeptoren können alle üblichen Säurebindungsmittel verwendet werden. Hierzu gehören vorzugsweise tertiäre Amine, Erdalkaliverbindungen, Ammoniumverbindungen und Alkaliverbindungen sowie entsprechende Gemische. Es können aber auch Zinkverbindungen verwendet werden. Es kommen z.B. als basische Verbindungen in Frage: Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Trimethylamin, Triethylamin, Tripropylamin, Triisopropylamin, Tributylamin, Triisobutylamin, Tri-sec.-butylamin, Tri-tert.-butylamin, Tribenzylamin, Tricyclohexylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropyl-p-aminopyridin, N-Methylpyrrolidon, N-Ethylpyrrolidon, N-Methylpiperidin, N-Ethylpiperidin, N-Methyl-pyrrolidin, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethylenimin, N-Ethylhexamethylenimin, Pyridin, Chinolin, α-Picolin, β-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N-Dimethylcyclohexylamin, 2,6-Lutidin, 2,4-Lutidin, Trifurfurylamin, Triethylendiamin.

Zweckmäßigerweise verwendet man den Säureakzeptor in einem Über- oder Unterschuß von bis zu 20%, bezogen auf den Ausgangsstoff II. Man kann jedoch auch den entstehenden Halogenwasserstoff durch Eingasen eines Inertgages, beispielsweise von Stickstoff, entfernen.

Die zur Umsetzung benötigten Ausgangsstoffe III werden im allgemeinen in einem Über- oder Unterschuß von bis zu 20%, bezogen auf den Ausgangsstoff II, eingesetzt. Man kann den Ausgangsstoff III jedoch auch direkt als Lösungsmittel verwenden. Mann kann jedoch auch den Ausgangsstoff III in einem der vorgenannten Verdünnungsmittel vorlegen und dann den Ausgangsstoff II und einen Säureakzeptor, gleichzeitig oder in beliebiger Reihenfolge, über zwei getrennte Zuführungen zugeben.

Zweckmäßigerweise wird das Verfahren zur Herstellung der neuen Verbindungen so durchgeführt, daß man den Ausgangsstoff II, gegebenenfalls in einem der vorgenannten Verdünnungsmittel vorlegt und dann den Ausgangsstoff III und einen Säureakzeptor gleichzeitig oder nacheinander zugibt. Mann kann jedoch auch den Ausgangsstoff III in einem der vorgenannten Verdünnungsmittel vorlegen und dann den Ausgangsstoff II und einen Säureakzeptor, gleichzeitig oder in beliebiger Reihenfolge, über zwei getrennte Zuführungen zugeben.

Die Umsetzung ist in vielen Fällen nach der Zugabe der Komponenten bereits abgeschlossen, andernfalls rührt man zu ihrer Beendigung noch 10 Minuten bis 10 Stunden bei −50 bis 150°C, vorzugsweise 0 bis 120°C, insbesondere 10 bis 50°C, nach.

Verwendet man ein Inertgas zur Entfernung des Halogenwasserstoffes, so rührt man zweckmäßigerweise 0,2 bis 10 Stunden bei 40 bis 100°C nach.

Aus dem Reaktionsgemisch wird der Endstoff I in üblicher Weise, z.B. nach Abdestillieren von

Lösungsmittel oder überschüssigem Ausgangsstoff III oder direkt durch Absaugen, isoliert. Der verbleibende Rückstand wird in diesem Fall zur Entfernung saurer Verunreinigungen mit Wasser bzw. verdünntem Alkali gewaschen und getrocknet. Im Falle von mit Wasser nicht mischbaren Verdünnungsmitteln kann man auch direkt das Reaktionsgemisch mit Wasser bzw. mit verdünntem Alkali extrahieren und dann trocknen und einengen. Man kann jedoch auch den Rückstand in einem mit Wasser nicht mischbaren Lösungsmittel lösen und wie beschrieben waschen. Die gewünschten Endstoffe fallen hierbei in reiner Form an, gegebenenfalls können sie durch Umkristallisation, Chromatographie oder Destillation gereinigt werden.

Synthesevorschrift 1

2,5-Dimethyl-3-(pyrid-3-yl-oxy)-2H-1,2,4,6-thiatriazin-1,1-dioxid (nicht beansprucht)

17,1 Teile β-Hydroxypyridin in 120 Teilen Methylenchlorid un 19,1 Teile N,N-Dimethylcyclohexylamin werden über 2 Zuführungen unter Rühren gleichzeitig innerhalb 15 Minuten bei 15 bis 20°C zu einer Lösung von 29,3 Teilen 2,5-Dimethyl-3-chlor-2H-1,2,4,6-thiatriazin-1,1-dioxid in 150 Teilen Methylenchlorid gegeben. Nach einer Stunde Rühren bie 25°C wird der Neiderschlag abgesaugt; das Filtrat wird mit Wasser, mit verdünnter Natriumcarbonatlösung gewaschen und dann über Magnesiumsulfat getrocknet. Nach dem Chromatographieren über neutralem Aluminiumoxid und Einengen unter vermindertem Druck werden 26 Teile (68,6% d. Th.) 2,5-Dimethyl-3-(pyrid-3-yl-oxy)-2H-1,2,4,6-thiatriazin-1,1-dioxid vom Fp. 137 bis 138°C erhalten.

Synthesevorschrift 2

6-Ethyl-3-methoxy-5-(5-chlorchinolyl-8-oxy)-6H-1,2,4,6-thiatriazin-1,1-dioxid (nicht beansprucht)

21,5 Teile 5-Chlor-8-oxychinolin in 150 Teilen 1,2-Dichlorethan und 12,4 Teile Triethylamin werden innerhalb 10 Minuten unter Rühren bei 20 bis 25°C gleichzeitig zu 22,6 Teilen 6-Ethyl-3-methoxy-5-chlor-6H-1,2,4,6-thiatriazin-1,1-dioxid in 70 Teilen 1,2-Dichlorethan gegeben. Das Reaktionsgemisch wird anschließend 3 Stunden bei 40°C gerührt, abgekühlt und nacheinander mit Wasser, 1 n Salzsäure und mit verdünnter Natriumcarbonatlösung gewaschen. Nach dem Trocknen über Magnesiumsulfat und Einengen unter vermindertem Druck werden 28,8 Teile (79% d. Th.) 2-Ethyl-5-methoxy-3-(5-chlorchinolyl-8-oxy)-6H-1,2,4,6-thiatriazin-1,1-dioxid vom Fp. 206 bis 207°C erhalten.

Synthesevorschrift 3

6-Methyl-3-methoxy-5-(1-methyl-pyridaz-6-on-3-yl-oxy)-6H-1,2,4,6-thiatriazin-1,1-dioxid (nicht beansprucht)

25,3 Teile 6-Methyl-3-methoxy-5-chlor-6H-1,2,4,6-thiatriazin-1,1-dioxid in 50 Teilen Essigester und eine Mischung von 13,9 Teilen 1-Methyl-3-hydroxypyridaz-6-on und 13,3 Teilen Dimethylanilin in 125 Teilen Essigester werden gleichmäßig über 2 Zuführungen innerhalb 20 Minuten bei 15 bis 20°C in eine Rührapparatur gegeben. Nach eineinhalb Stunden Rühren bei 25°C wird das Reaktionsgemisch abgesaugt, mit 30 Teilen Essigester und dann mit Wasser sowie mit verdünnter Natriumcarbonatlösung gewaschen. Nach dem Trocknen unter vermindertem Druck werden 22,5 Teile (62% d. Th.) 2-Methyl-5-methoxy-3-(1-methyl-pyridaz-6-on-3-yl-oxy)-6H-1,2,4,6-thiatriazin-1,1-dioxid vom Fp. 206 bis 210°C erhalten.

Entsprechend den o.a. Beispielen werden die in der Tabelle angeführten Verbindungen erhalten.

| Verbindung Nr. | n | R | Fp [°C] $n_D^{25}$ |
|---|---|---|---|
| 1 | 0 | Pyrid-2-yl | 138—141 |
| 2 | 0 | Pyrid-3-yl | 165—168 |
| 3 | 0 | Pyrid-3-yl | |
| 4 | 0 | Pyrid-4-yl | 120 |
| 5 | 0 | 3-Methylpyrid-2-yl | |
| 6 | 0 | 3-Methylpyrid-4-yl | |
| 7 | 0 | 3-Methylpyrid-5-yl | |
| 8 | 0 | 3-Methylpyrid-6-yl | |
| 9 | 0 | 2-Methylpyrid-3-yl | |
| 10 | 0 | 2-Methylpyrid-4-yl | |

| Verbindung Nr. | n | R | Fp [°C] $n_D^{25}$ |
|---|---|---|---|
| 11 | 0 | 2-Methylpyrid-5-yl | 155—158 |
| 12 | 0 | 2-Methylpyrid-6-yl | 148—150 |
| 13 | 0 | 4-Methylpyrid-2-yl | |
| 14 | 0 | 4-Methylpyrid-3-yl | |
| 15 | 0 | 2-Chlorpyrid-4-yl | |
| 16 | 0 | 2-Chlorpyrid-5-yl | |
| 17 | 0 | 2-Chlorpyrid-6-yl | 139—142 |
| 18 | 0 | 3-Chlorpyrid-2-yl | |
| 19 | 0 | 3-Chlorpyrid-4-yl | |
| 20 | 0 | 3-Chlorpyrid-5-yl | 131—132 |
| 21 | 0 | 3-Chlorpyrid-6-yl | 141—146 |
| 22 | 0 | 4-Chlorpyrid-2-yl | |
| 23 | 0 | 4-Chlorpyrid-3-yl | |
| 24 | 0 | 3,5-Dichlorpyrid-2-yl | |
| 25 | 0 | 3-Chlor-5-trifluormethylpyrid-2-yl | |
| 26 | 0 | 2-Trifluormethylpyrid-3-yl | |
| 27 | 0 | 2-Trifluormethylpyrid-4-yl | |
| 28 | 0 | 2-Trifluormethylpyrid-5-yl | |
| 29 | 0 | 2-Trifluormethylpyrid-6-yl | |
| 30 | 0 | 3-Trifluormethylpyrid-2-yl | |
| 31 | 0 | 3-Trifluormethylpyrid-4-yl | |
| 32 | 0 | 3-Trifluormethylpyrid-5-yl | |
| 33 | 0 | 3-Trifluormethylpyrid-6-yl | |
| 34 | 0 | 4-Trifluormethylpyrid-2-yl | |
| 35 | 0 | 4-Trifluormethylpyrid-3-yl | |
| 36 | 0 | 2-Nitropyrid-3-yl | |
| 37 | 0 | 2-Nitropyrid-4-yl | |
| 38 | 0 | 2-Nitropyrid-5-yl | |
| 39 | 0 | 2-Nitropyrid-6-yl | |
| 40 | 0 | 3-Nitropyrid-2-yl | |
| 41 | 0 | 3-Nitropyrid-4-yl | |

| Verbindung Nr. | n | R | Fp [°C] $n_D^{25}$ |
|---|---|---|---|
| 42 | 0 | 3-Nitropyrid-5-yl | |
| 43 | 0 | 3-Nitropyrid-6-yl | 147—150 |
| 44 | 0 | 4-Nitropyrid-2-yl | |
| 45 | 0 | 4-Nitropyrid-3-yl | |
| 46 | 0 | 3,5-Diiodpyrid-2-yl | |
| 47 | 0 | Pyrimidin-2-yl | |
| 48 | 0 | Pyrimidin-4-yl | |
| 49 | 0 | Pyrimidin-5-yl | |
| 50 | 0 | 2-Methylpyrimidin-4-yl | |
| 51 | 0 | 2,6-Dimethylpyrimidin-4-yl | |
| 52 | 1 | Pyrid-2-yl | 105—108 |
| 53 | 1 | Pyrid-3-yl | |
| 54 | 1 | Pyrid-4-yl | |
| 55 | 0 | 4-Methylpyrimidin-2-yl | |
| 56 | 0 | 2-Isopropyl-6-methyl-pyrimidin-4-yl | 88—91 |
| 57 | 0 | Pyrazin-2-yl | |
| 58 | 0 | 5-Chlor-pyrazin-2-yl | |
| 59 | 0 | 5-Methyl-pyrazin-2-yl | |
| 60 | 1 | 1-Methylpiperazin-4-yl | |
| 61 | 1 | 1,2,3-Thiadiazol-4-yl | 144—147 |
| 62 | 1 | 1,2,3-Thiadiazol-5-yl | |
| 63 | 1 | Pyrid-2-yl | |

Die Verbindungen der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert

werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. 20 Gewichtsteile der Verbindung Nr. 1 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-mono-ethanolamid, 5 Gewichtsteilen Calciumsalz der Dodecyl-benzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

II. 20 Gewichtsteile der Verbindung Nr. 11 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 56 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

IV. 3 Gewichtsteile der Verbindung Nr. 43 werden mit 97 Gewichtsteilen feinteiligem Kaolin vermischt. Man erhält auf diese Weise ein Stäubemittel, das 3 Gewichtsprozent des Wirkstoffs enthält.

Die Applikation der Wirkstoffe bzw. der Mittel kann im Vorauflaufverfahren oder bei Nachauflaufanwendung erfolgen. Sind die Wirkstoffe für die Kulturpflanze weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit und Wachstumsstadium 0,1 bis 10 kg/ha und mehr, vorzugsweise 0,5 bis 5 kg/ha, wobei sich die höheren Dosen besonders zur totalen Bekämpfung von Vegetationen eignen.

Die herbizide Wirkung von Verbindungen der Formel I wird durch Gewächshausversuche gezeigt:

Als Kulturgefäße dienen Plastikblumentöpfe mit 300 $cm^3$ Inhalt und lehmigem Sand mit etwa 1,5% Humus als Substrat. Die Samen der Testpflanzen werden nach Arten getrennt flach eingesät. Unmittelbar danach erfolgt bei Vorauflaufbehandlung das Aufbringen der herbiziden Mittel auf die Erdoberfläche. Die Wirkstoff-Formulierungen werden hierzu in Wasser als Verteilungsmittel suspendiert oder emulgiert und mittels fein verteilender Düsen gespritzt. Bei dieser Applikationsmethode beträgt die Aufwandmenge je nach Wirkstoff 1,0, 2,0 oder 3,0 kg Wirkstoff/ha. Nach dem Aufbringen der Mittel beregnet man die Gefäße leicht, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu

einer Wuchshöhe von 3 bis 15 cm an und behandelt sie danach. Dabei handelt es sich um entweder direkt in die zu behandelnden Gefäße gesäte Pflanzen oder um separat angezogene Pflanzen, die in die Gefäße umgepflanzt wurden. Die Aufwandmenge beträgt 3,0 kg Wirkstoff/ha. Die Abdeckung unterbleibt nach der Blattbehandlung.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 0 keine Schädigung oder normaler Auflauf und 100 kein Aufgang der Pflanzen bzw. völlige Zerstörung zumindest der oberirdischen Teile.

Bei den Testpflanzen handelt es sich um Pflanzen der Arten:

Centaurea cyanus (Kornblume), Chenopodium album (Weißer Gänsefuß), Cyperus esculentus (Erdmantel), Datura stramonium (Gemeiner Stechapfel), Echinochloa crus-galli (Hühnerhirse), Galium aparine (Klettenlabkraut), Gossypium hirsutum (Baumwolle), Ipomoea spp. (Prunkwindearten), Lolium multiflorum (Ital. Rayegras), Mentha piperita (Pfefferminze), Nicandra physaloides (Giftbeere), Oryza sativa (Reis), Sinapis alba (Weißer Senf), Solanum nigrum (Schwarzer Nachtschatten), Viola spp. (Stiefmütterchen), Zea Mays (Mais).

In diesen Versuchen haben beispielsweise die Verbindungen Nr. 12 und 56 bei Vorauflaufanwendung von 3,0 kg Wirkstoff/ha eine gute herbizide Wirkung.

Im Nachauflaufverfahren bekämpfen beispielsweise die Verbindungen Nr. 43 und 56 bei einer Aufwandmenge von 3,0 kg/ha unerwünschte Pflanzen sehr erfolgreich. Zur selektiven Unkrautbekämpfung sind beispielsweise die Verbindungen Nr. 1, 11 und 61 mit 1,0 kg Wirkstoff/ha und die Verbindung Nr. 56 mit 2,0 kg/ha geeignet. Die Verbindung Nr. 20 bekämpft mit 0,5 kg Wirkstoff/ha unerwünschte Pflanzen in Reis und Mais ohne bzw. mit geringer Schädigung der Kulturpflanzen.

In Anbetracht der guten Verträglichkeit der Wirkstoffe und der Vielseitigkeit der Applikationsmethoden können die erfindungsgemäßen Verbindungen oder diese enthaltende Mittel außer bei den in den Gewächshausversuchen getesteten Nutzpflanzen noch in einer weiteren großen Zahl von Kulturpflanzen zur Beseitigung unerwünschten Pflanzenwuchses eingesetzt werden.

In Betracht kommen beispielsweise die folgenden Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Camellia sinensis | Teestrauch |
| Carthamus tinctorius | Saflor — Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Daucus carota | Möhre |
| Elaeis guineensis | Ölpalme |

| Botanischer Name | Deutscher Name |
|---|---|
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersiocon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |

| Botanischer Name | Deutscher Name |
|---|---|
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Vaccinium carymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Verbindungen der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem kann es von Nutzen sein, die erfindungsgemäßen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Erährungs- oder Spurenelementmängeln eingesetzt werden. Zur Aktivierung der herbiziden Wirkung können auch Netz- und Haftmittels sowie nicht-phytotoxische Öle und Ölkonzentrate zugesetzt werden.

**Patentansprüche**

1. 1,2,4,6-Thiatriazin-1,1-dioxid-ether der Formel I

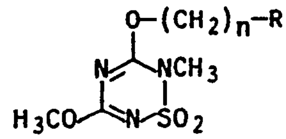

(I)

in der

R einen gegebenenfalls durch Alkyl mit 1 bis 4 Kohlenstoffatomen, Trifluormethyl, Nitro oder Halogen substituierten 6-gliedrigen aromatischen heterocyclischen Rest mit 1 oder 2 Stickstoffatomen oder 1,2,3-Thiadiazol und

n 0 oder 1 bedeuten.

2. 1,2,4,6-Thiatriazin-1,1-dioxid-ether der Formel I gemäß Anspruch 1, dadurch gekennzeichnet, daß R einen gegebenenfalls durch Alkyl mit 1 bis 4 Kohlenstoffatomen, Nitro oder Halogen substituierten Pyridylrest und n 0 oder 1 bedeuten.

3. 1,2,4,6-Thiatriazin-1,1-dioxid-ether der Formel I gemäß Anspruch 1, dadurch gekennzeichnet, daß R einen gegebenenfalls durch Alkyl mit 1 bis 4 Kohlenstoffatomen oder Halogen substituierten Pydrimidinyl- oder 1,2,3-Thiadiazolylrest und n 0 oder 1 bedeuten.

4. 1,2,4,6-Thiatriazin-1,1-dioxid-ether der Formel I gemäß Anspruch 1, dadurch gekennzeichnet, daß R einen Pyridyl-, Methylpyridyl-, Nitropyridyl-, 1,2,3-Thiadiazolyl- oder einen 2-Isopropyl-6-methyl-pyrimidinylrest und n 0 oder 1 bedeuten.

5. 1,2,4,6-Thiatriazin-1,1-dioxid-ether der Formel I gemäß Anspruch 1, dadurch gekennzeichnet, daß R einen gegebenenfalls durch Nitro, Halogen oder Alkyl mit 1 bis 4 Kohlenstoffatomen substituierten Pyridylrest und n 0 bedeuten.

6. Verfahren zur Herstellung von 1,2,4,6-Thiatriazin-1,1-dioxid-ethern der Formel I gemäß Anspruch 1, dadurch gekennzeichnet, daß man ein substituiertes 1,2,4,6-Thiatriazin-1,1-dioxid der Formel II

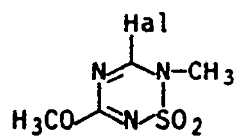

(II)

in der Hal für Halogen steht, mit einer Verbindung der Formel III

$$HO—(CH_2)_n—R \qquad (III)$$

oder einem Alkali-, Erdalkali- oder Ammoniumsalz einer Verbindung der Formel III, gegebenenfalls in einem inerten organischen Lösungsmittel und gegebenenfalls in Anwesenheit eines Säureakzeptors umsetzt.

7. Herbizid, enthaltend einen 1,2,4,6-Thiatriazin-1,1-dioxid-ether der Formel I gemäß Anspruch 1.

8. Herbizid, enthaltend inerte Zusatzstoffe und einen 1,2,4,6-Thiatriazin-1,1-dioxid-ether der Formel I gemäß Anspruch 1.

9. Verfahren zur Bekämpfung unerwünschten Pflanzenwuches, dadurch gekennzeichnet, daß man die Pflanzen und/oder ihren Standort mit einer herbizid wirksamen Menge eines 1,2,4,6-Thiatriazin-1,1-dioxid ethers der Formel I gemäß Anspruch 1 behandelt.

**Revendications**

1. Ethers-1,1-dioxydes de 1,2,4,6-thiatriazines de formule I

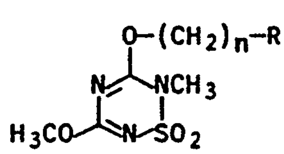

(I)

dans laquelle

R représente un radical hétérocyclique aromatique à 6 chaînons et 1 ou 2 atomes d'azote, éventuellement substitué par des groupes alkyle en C 1—C 4, trifluorométhyle, nitro ou des halogènes, ou le 1,2,3-thiadiazole, et

n est égal à 0 ou 1.

2. Ethers-1,1-dioxydes de 1,2,4,6-thiatriazines de formule I selon la revendication 1, caractérisés en ce que R représente un radical pyridyle éventuellement substitué par des groupes alkyle en C 1—C 4, nitro ou des halogènes et n est égal à 0 ou 1.

3. Ethers-1,1-dioxydes de 1,2,4,6-thiatriazines de formule I selon la revendication 1, caractérisés en ce que R représente un radical pyrimidinyle ou 1,2,3-thiadiazolyle éventuellement substitué par des groupes alkyle en C 1—C 4 ou des halogènes et n est égal à 0 ou 1.

4. Ethers-1,1-dioxydes de 1,2,4,6-thiatriazines de formule I selon la revendication 1, caractérisés en ce que R représente un radical pyridyle, méthylpyridyle, nitropyridyle, 1,2,3-thiadiazolyle ou un radical 2-isopropyl-6-méthyl-pyrimidinyle et n est égal à 0 ou 1.

5. Ethers-1,1-dioxydes de 1,2,4,6-thiatriazines de formule I selon la revendication 1, caractérisés en ce que R représente un radical pyridyle éventuellement substitué par des groupes nitro, des halogènes ou des groupes alkyle en C 1—C 4 et n est égal à 0.

6. Procédé de préparation des éthers-1,1-dioxydes de 1,2,4,6-thiatriazines de formule I selon la revendication 1, caractérisé en ce que l'on fait réagir un 1,1-dioxyde de 1,2,4,6-thiatriazine substitué de formule II

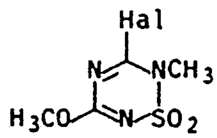

(II)

dans laquelle Hal représente un halogène, avec un composé de formule III

$$HO—(CH_2)_n—R \quad (III)$$

ou un sel de métal alcalin, alcalino-terreux ou d'ammonium d'un composé de formule III, éventuellement dans un solvant organique inerte et éventuellement en présence d'un accepteur d'acide.

7. Produit herbicide contenant un éther-1,1-dioxyde de 1,2,4,6-thiatriazine de formule I selon la revendication 1.

8. Produit herbicide contenant des additifs inertes et un éther-1,1-dioxyde de 1,2,4,6-thiatriazine de formule I selon la revendication 1.

9. Procédé pour combattre les croissances de végétaux indésirables, caractérisé en ce que l'on traite les végétaux et/ou leur habitat par une quantité herbicide efficace d'un éther-1,1-dioxyde de 1,2,4,6-thiatriazine de formule I selon la revendication 1.

## Claims

1. A 1,2,4,6-thiatriazine 1,1-dioxide ether of the formula I

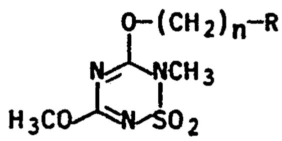

(I)

2. A 1,2,4,6-thiatriazine 1,1-dioxide ether of the formula I as claimed in claim 1, wherein R is a pyridyl radical which is unsubstituted or substituted by alkyl of 1 to 4 carbon atoms, nitro or halogen and n is 0 or 1.

3. A 1,2,4,6-thiatriazine 1,1-dioxide ether of the formula I as claimed in claim 1, wherein R is a pyrimidinyl or 1,2,3-thiadiazolyl radical which is unsubstituted or substituted by alkyl of 1 to 4 carbon atoms or by halogen, and n is 0 or 1.

4. A 1,2,4,6-thiatriazine 1,1-dioxide ether of the formula I as claimed in claim 1, wherein R is a pyridyl, methylpyridyl, nitropyridyl, 1,2,3-thiadiazolyl or 2-isopropyl-6-methylpyrimidinyl radical and n is 0 or 1.

5. A 1,2,4,6-thiatriazine 1,1-dioxide ether of the formula I as claimed in claim 1, wherein R is pyridyl which is unsubstituted or substituted by nitro, halogen or alkyl of 1 to 4 carbon atoms, and n is 0.

6. A process for the preparation of a 1,2,4,6-thiatriazine 1,1-dioxide ether of the formula I as claimed in claim 1, wherein a substituted 1,2,4,6-thiatriazine 1,1-dioxide of the formula II

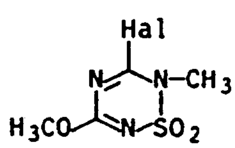

(II)

where Hal is halogen, is reacted with a compound of the formula III

$$HO—(CH_2)_n—R \quad (III)$$

or with an alkali metal salt, alkaline earth metal salt or ammonium salt of a compound of the formula III, in the presence or absence of an inert organic solvent and in the presence or absence of an acid acceptor.

7. A herbicide containing a 1,2,4,6-thiatriazine 1,1-dioxide ether of the formula I as claimed in claim 1.

8. A herbicide containing inert additives and a 1,2,4,6-thiatriazine 1,1-dioxide ether of the formula I as claimed in claim 1.

9. A process for controlling the growth of undesirable plants, wherein the plants and/or their location are treated with a herbicidally effective amount of a 1,2,4,6-thiatriazine 1,1-dioxide ether of the formula I as claimed in claim 1.